# EUROPEAN PATENT APPLICATION

(11) **EP 2 239 274 A1**
(43) Date of publication of application: **13.10.2010**
(21) Application number: 10162295.9
(22) Date of filing: 02.01.2003
(51) Int. Cl.: C07K 19/00, A61K 47/48, A61K 51/10, A61P 35/00

(54) **New methods for diagnosis and treatment of tumours**

(30) Priority: 25.02.2002 US 358702 P; 03.01.2002 EP 02000315
(62) Divisional of application: 03726977.6
(71) Applicant: Bayer Schering Pharma AG, 13353 Berlin (DE)
(72) Inventor: Hilger, Christoph-Stephan, 13503 Berlin (DE); Berndorff, Dietmar, 13467 Berlin (DE); Dinkelborg, Ludger, 13465, Berlin (DE); Moosmayer, Dieter, 13353, Berlin (DE); Neri, Giovanni, 52100, Siena (IT)
(74) Representative: Weiss, Wolfgang

(57) **Abstract**

The present invention relates to new methods for diagnosis and treatment of tumours, using novel peptides for binding radionuclides.

## Description

### Field of the invention

The present invention relates to new methods for diagnosis and treatment of tumours, using novel peptides for binding radionuclides.

### Brief description of the background art

Tumours cannot gain more than a certain weight without the formation of new blood vessels (angiogenesis), and a correlation between microvessel density and tumour invasiveness has been reported for a number of tumours (Folkman (1995), Nature Med., 1, 27 - 31). Moreover, angiogenesis is involved in the majority of ocular disorders which result in loss of vision (Lee et al., Surv. Ophthalmol. 43, 245 - 269 (1998); Friedlander, M. et al., Proc. Natl. Acad. Sci. U.S.A. 93, 9764 - 9769 (1996)). Molecules capable of selectively targeting markers of angiogenesis would create clinical opportunities for the diagnosis and therapy of tumours and other diseases characterised by vascular proliferation, such as diabetic retinopathy and age-related macular degeneration. Markers of angiogenesis are expressed in the majority of aggressive solid tumours in association with tumoural vessels and should therefore be readily accessible to specific binders injected intravenously (Pasqualini et al., (1997), Nature Biotechnol., 15, 542 - 546; Neri et al. (1997), Nature Biotechnol., 15, 1271 - 1275). Targeted occlusion of the neovasculature may result in tumour infarction and collapse (O'Reilly et al. (1996), Nature Med., 2, 689 - 692; Huang et al. (1997), Science, 275, 547 - 550).

The ED-B domain of fibronectin, a sequence of 91 amino acids identical in mouse, rat and human, which is inserted by alternative splicing into the fibronectin molecule, specifically accumulates around neo-vascular structures (Castellani et al. (1994), Int. J. Cancer 59, 612 - 618) and could represent a target for molecular intervention. Indeed, it has recently been shown with fluorescent techniques that anti-ED-B single-chain Fv antibody fragments (scFv) accumulate selectively around tumoural blood vessels of tumour-bearing mice, and that antibody affinity appears to dictate targeting performance (Neri et al. (1997), Nature Biotechnol., 15, 1271 - 1275; WO 97/45544).

Furthermore, antibodies and antibody fragments specific for binding the ED-B domain of fibronectin with a sub-nanomolar dissociation constant as well as radiolabeled derivatives thereof are described in WO 99/58570. The biodistribution of one of these high-affinity human antibody fragments, the ¹²⁵I labelled antibody fragment called L19, was already investigated in tumour-bearing mice (Tarli et al., Blood, Vol. 94, No. 1 (1999), p. 192 - 198). Radiolabeled conjugates comprising L19-antibodies and their use for the detection and treatment of angiogenesis are disclosed in WO 01/62800.

The recombinant production of functionalized single-chain Fv antibody fragments binding to the ED-B domain of the B-isoform of fibronectin in *Pichia pastoris* has already been described (Marty et al., Protein Expression and Purification 21, 156 - 164 (2001)).

Further, radiolabeling of scFv antibody fragments with ^{99m}Tc through a C-terminal cysteinyl peptide was described by George et al., Proc. Natl. Acad. Sci. USA, Vol. 92 pp. 8358 - 8362, 1995, and by Verhaar et al., J. Nuc. Med., Vol. 37(5), pp. 868 - 872, 1996.

However, there is still a clinical need for providing antibody fragments that have improved pharmacokinetic properties, and that can easily be labeled with radioisotopes of e.g. Technetium or Rhenium, since these radionuclides are particular well suited for radiopharmaceuticals.

### Object of the invention

It is therefore an object of the invention to provide antibody fragments that have improved pharmacokinetic properties, particularly target specifity and/or in vivo stability, and that can easily bind radioisotopes e.g. of Technetium or Rhenium.

### Summary of the invention

The present invention describes compounds comprising a peptide comprising
- aa): the sequence of the antigen-binding site for the extra domain B (ED-B) of fibronectin comprising complementarity- determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to Seq. Id. No. 1,
- ab): the sequence of the antigen-binding site for the extra domain B (ED-B) of fibronectin comprising complementarity- determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to Seq. Id. No. 1, or
- ac): the sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
and
- ba): an amino acid sequence Xaa₁-Xaa₂-Xaa₃-Cys (Seq. Id. No. 2), wherein Xaa₁, Xaa₂, and Xaa₃ each independently represent any naturally occuring amino acid or
- bb): an amino acid sequence Xaa₁-Xaa₂-Xaa₃-Cys-Xaa₄ (Seq. Id. No. 3), wherein Xaa₁, Xaa₂, Xaa₃, and Xaa₄ each independently represent any naturally occuring amino acid or
- bc): an amino acid sequence (His)ₙ (Seq. Id. No. 4), wherein n stands for an integer from 4 to 6, wherein the C-terminus of aa), ab) or ac) is bound to the N- terminus of one of the sequences Seq. Id. No. 2, Seq. Id. No. 3 or Seq. Id. No. 4 via a peptide bond.

The compounds are preferably single chain antibody fragments, particularly scFv fragments. Further, the compounds are preferably conjugated to a radioisotope, e.g. a radioisotope of Technetium, such as ^{94m}Tc, ^{99m}Tc Rhenium, such as ¹⁸⁶Re, ¹⁸⁸Re, or other isotopes, such as ²⁰³Pb, ⁶⁷Ga, ⁶⁸Ga, ⁴³Sc, ⁴⁴Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ⁷²As and ¹⁸F.

The present invention also describes a pharmaceutical composition comprising the above compound as active agent together with physiologically acceptable adjuvants, diluents and/or carriers.

The present invention also describes the use of a peptide comprising
- aa): the sequence of the antigen-binding site for the extra domain B (ED-B) of fibronectin comprising complementarity- determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to SEQ Id. No. 1;
- ab): the sequence of the antigen-binding site for the extra domain B(ED-B) of fibronectin comprising complementarity- determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to SEQ Id. No. 1;
- ac): a sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
and
- ba): an amino acid sequence Xaa₁-Xaa₂-Xaa₃-Cys (Seq. Id. No. 2), wherein Xaa₁, Xaa₂, and Xaa₃ each independently represent any naturally occuring amino acid or
- bb): an amino acid sequence Xaa₁-Xaa₂-Xaa₃-Cys-Xaa₄ (Seq. Id. No. 3), wherein Xaa₁, Xaa₂, Xaa₃, and Xaa₄ each independently represent any naturally occuring amino acid or
- bc): an amino acid sequence (His)ₙ (Seq. Id. No. 4), wherein n stands for an integer from 4 to 6, wherein the C-terminus of aa), ab) or ac) is bound to the N- terminus of one of the sequences Seq. Id. No. 2, Seq. Id. No. 3 or Seq. Id. No. 4 via a peptide bond,
for binding a radioisotope, e.g. a radioisotope of Technetium or Rhenium.

The antibody fragment L19 is defined by the following sequence (Seq. Id. No.1):

**(VH)**

| | | |
|---|---|---|
| E V Q L L E S G G G | L V Q P G G S L R L | S C A A S G F T F S |
| S F S M S W V R Q A | P G K G L E W V S S | I S G S S G T T Y Y |
| A D S V K G R F T I | S R D N S K N T L Y | L Q M N S L R A E D |
| T A V Y Y C A K P F | P Y F D Y W G Q G T | L V T V S S |
| (Linker) | | |
| G D G S S G G S G G | A S T G | |
| (VL) | | |
| E I V L T Q S P G T | L S L S P G E R A T | L S C R A S Q S V S |
| S S F L A W Y Q Q K | P G Q A P R L L I Y | Y A S S R A T G I P |
| D R F S G S G S G T | D F T L T I S R L E | P E D F A V Y Y C Q |
| Q T G R I P P T F G | Q G T K V E I K | |

A deletion, insertion and/or substitution of up to 30 amino acids is a deletion, insertion and/or substitution of 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids of Seq. Id. No.1. However, within the complementarity-determining regions (CDRs) of the claimed peptide, e.g. the peptide of Seq. Id. No. 1, a variation that is a deletion, insertion and/or substitution of amino acids (aa) should not exceed the maximum variations defined in Table 1 below (HCDR: CDR of the heavy chain; LCDR: CDR of the light chain).

**Table 1**

| Region | CDR length (aa) | Sequence | Maximum (preferred) variations in sequence positions |
|---|---|---|---|
| HCDR1 | 5 | S F S M S | 3 (2,1) |
| HCDR2 | 17 | S I S G S S G T T Y Y A D S V K G | 8 (7,6,5,4,3,2,1) |
| HCDR3 | 7 | P F P Y F D Y | 5 (4,3,2,1) |
| LCDR1 | 12 | R A S Q S V S S S F L A | 6 (5,4,3,2,1) |
| LCDR2 | 7 | Y A S S R A T | 4(3,2,1) |
| LCDR3 | 10 | C Q Q T G R I P P T | 6(5,4,3,2,1) |

The CDRs were defined according to E. A. Kabat et al., "Sequences of Proteins of Immunological Interest", U.S. Department of Health and Human Services, National Institutes for Health, Bethesda, MD, 5th Edition, 1991.

Preferred are peptides comprising a sequence according to Seq. Id. No. 1 (L19) or a variation of Seq. Id. No. 1 that is a deletion, insertion and/or substitution of up to 20 amino acids.

A peptide comprising a variation of the CDR sequences as shown in Table 1 and particularly a variation of Seq. Id. No. 1 that is a deletion, insertion and/or substitution, and which has the same function as the peptide according to Seq. Id. No. 1, is defined as a peptide that binds to the ED-B domain of fibronectin with a dissociation constant K_{d} that is in the subnanomolar range (i.e. less than 10⁻⁹), measured with a BlAcore (see WO99/58570, Example 2 and Table 2).

Preferred amino acid sequences Xaa₁-Xaa₂-Xaa₃-Cys (Seq. Id. No. 2) are the sequences Gly-Gly-Gly-Cys (Seq. Id. No. 5) and Gly-Cys-Gly-Cys (Seq. Id. No. 6). Most preferred is the sequence Gly-Gly-Gly-Cys (Seq. Id. No. 5).

Preferred amino acid sequences Xaa₁-Xaa₂-Xaa₃-Cys-Xaa₄ (Seq. Id. No. 3) are the sequences Gly-Gly-Gly-Cys-Ala (Seq. Id. No. 7) and Gly-Cys-Gly-Cys-Ala (Seq. Id. No. 8). Most preferred is the sequence Gly-Gly-Gly-Cys-Ala (Seq. Id. No. 7).

In compounds comprising an amino acid sequence (His)ₙ (Seq. Id. No. 4), those compounds wherein n stands for the integer 6 are preferred.

Preferred radioisotopes of Technetium or Rhenium are the isotopes ^{94m}Tc, ^{99m}Tc, ¹⁸⁶Re and ¹⁸⁸Re. Most preferred is the radioisotope ^{99m}Tc.

### Detailed Description of the Invention

The single-chain antibody fragment L19 (Seq. Id. No. 1) was previously labeled with ¹²⁵I to investigate the biodistribution of this compound in tumour-bearing mice (Tarli et al., Blood, Vol. 94, No. 1 (1999), p. 192 - 198). The results show that a selective targeting of tumoural blood vessels in vivo may be accomplished. Surprisingly however, it was found that the pharmacokinetic properties of the single-chain antibody fragment L19 may be substantially improved when it is conjugated to a peptide ba), bb) or bc) and labelled with radioisotopes of Technetium or Rhenium. The isotope ^{99m}Tc is the radiolabel of choice for routine clinical SPECT due to its radiochemical properties (easily available through a ⁹⁹Mo/⁹⁹mTc generator, emits single gamma-photons of 140 KeV, has high photon flux, and decays with a half-life of 6 hours) and due to its cost-effectiveness. For therapeutic applications, labeling with the chemically analogous isotopes ¹⁸⁶Re and ¹⁸⁸Re is especially preferred (Hsieh, B.T., et al., Nucl. Med. Biol., 1999, 26(8), 967-972; 973-976, Zamora, P.O., et al., Anticancer Res., 1997, 17(3B), 1803-1838).

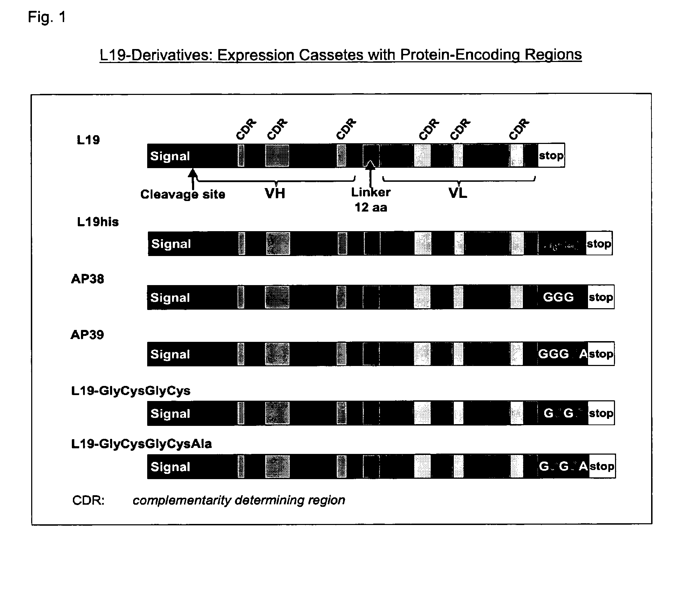

The peptides of the present invention are derivatives of the recombinant scFv antibody L19 (Seq. Id. No. 1) against the extracellular ED-B domain of fibronectin and were produced via genetic engineering according to Fig. 1. The following peptides were produced:

**L19 (Seq. Id. No. 1)**

| | |
|---|---|
| L19His: | |
| 1 | EVQLLESGGG LVQPGGSLRL SCAASGFTFS SFSMSWVRQA PGKGLEWVSS |
| 51 | ISGSSGTTYY ADSVKGRFTI SRDNSKNTLY LQMNSLRAED TAVYYCAKPF |
| 101 | PYFDYWGQGT LVTVSSGDGS SGGSGGASEI VLTQSPGTLS LSPGERATLS |
| 151 | CRASQSVSSS FLAWYQQKPG QAPRLLIYYA SSRATGIPDR FSGSGSGTDF |
| 201 | TLTISRLEPE DFAVYYCQQT GRIPPTFGQG TKVEIKAAAL EHHHHHH |

**(Seq. Id. No. 9)**

| | |
|---|---|
| AP38: | |
| 1 | EVQLLESGGG LVQPGGSLRL SCAASGFTFS SFSMSWVRQA PGKGLEWVSS |
| 51 | ISGSSGTTYY ADSVKGRFTI SRDNSKNTLY LQMNSLRAED TAVYYCAKPF |
| 101 | PYFDYWGQGT LVTVSSGDGS SGGSGGASEI VLTQSPGTLS LSPGERATLS |
| 151 | CRASQSVSSS FLAWYQQKPG QAPRLLIYYA SSRATGIPDR FSGSGSGTDF |
| 201 | TLTISRLEPE DFAVYYCQQT GRIPPTFGQG TKVEIKGGGC |

**(Seq. Id. No. 10)**

| | |
|---|---|
| AP39: | |
| 1 | EVQLLESGGG LVQPGGSLRL SCAASGFTFS SFSMSWVRQA PGKGLEWVSS |
| 51 | ISGSSGTTYY ADSVICGRFTI SRDNSKNTLY LQMNSLRAED TAVYYCAKPF |
| 101 | PYFDYWGQGT LVTVSSGDGS SGGSGGASEI VLTQSPGTLS LSPGERATLS |
| 151 | CRASQSVSSS FLAWYQQKPG QAPRLLIYYA SSRATGIPDR FSGSGSGTDF |
| 201 | TLTISRLEPE DFAVYYCQQT GRIPPTFGQG TKVEIKGGGC A |

**(Seq. Id. No. 11)**

| | |
|---|---|
| L19-GlyCysGlyCys: | |
| 1 | EVQLLESGGG LVQPGGSLRL SCAASGFTFS SFSMSWVRQA PGKGLEWVSS |
| 51 | ISGSSGTTYY ADSVKGRFTI SRDNSKNTLY LQMNSLRAED TAVYYCAKPF |
| 101 | PYFDYWGQGT LVTVSSGDGS SGGSGGASEI VLTQSPGTLS LSPGERATLS |
| 151 | CRASQSVSSS FLAWYQQKPG QAPRLLIYYA SSRATGIPDR FSGSGSGTDF |
| 201 | TLTISRLEPE DFAVYYCQQT GRIPPTFGQG TKVEIKGCGC |

**(Seq. Id. No. 12)**

| | |
|---|---|
| L19-GlycysGlycysAla: | |
| 1 | EVQLLESGGG LVQPGGSLRL SCAASGFTFS SFSMSWVRQA PGKGLEWVSS |
| 51 | ISGSSGTTYY ADSVKGRFTI SRDNSKNTLY LQMNSLRAED TAVYYCAKPF |
| 101 | PYFDYWGQGT LVTVSSGDGS SGGSGGASEI VLTQSPGTLS LSPGERATLS |
| 151 | CRASQSVSSS FLAWYQQKPG QAPRLLIYYA SSRATGIPDR FSGSGSGTDF |
| 201 | TLTISRLEPE DFAVYYCQQT GRIPPTFGQG TKVEIKGCGC A |
| (Seq. Id. No. 13) | |

The production of the peptides is described in detail in the following examples (see "Experimental").

The antibody fragment L19 was originally produced by expression in E. coli (see WO 99/58570). However, for the large-scale production of scFv antibody fragments, this expression system was found to be unsatisfying. Another expression system, a yeast expression system, particularly a *Pichia pastoris* expression system, was tested. The present inventors found that yeast, e.g. *Pichia pastoris* is generally capable for expression of a highly bioactive antibody fragment, e.g. the fragment AP39, but a high yield expression with up to 250 mg antibody fragment per liter culture, which is necessary for an economical production of a biopharmaceutical, could only be reached by a constitutive expression vector (e.g. pGAP), and not with a methanol inducible vector (e.g. pPIC9K) An additional advantage of this constitutive expression system is its simplified and robust fermentation procedures compared to an inducible yeast expression. Unexpectedly, the present inventors found that a proper signal sequence processing of the antibody fragment, e.g. the fragment AP39 was observed only when an expression cassette was used in which the N-terminus of the fragment was directly fused to the Kex2-cleavage site from the alpha-signal sequence.

The peptides are suitable for diagnostic and therapeutic applications, particularly for the diagnosis and therapy of invasive tumours and tumour metastases. Preferred diagnostic applications are SPECT (Single Photon Emission Computed Tomography) and PET (Positron Emission Tomography).

The peptides described above are particularly well suited for labeling radioisotopes as described above, e.g. radioisotopes of Technetium and Rhenium, preferably the radionuclides ^{94m}Tc, ^{99m}Tc, ¹⁸⁶Re, and ¹⁸⁸Re. For labeling the peptides, the peptides are first reduced with an appropriate reducing agent like e.g. stannous chloride or Tris(2-carboxyethyl)phosphine (TCEP). The resulting reduced peptides exhibit SH-groups that can react with ^{99m}Tc generator eluate or ¹⁸⁸Re generator eluate and stannous chloride to the compounds of the present invention (for details, see the experimental examples below). Indirect labeling is performed by pre-conjugating a chelating ligand and subsequent complexation of radioisotopes, such as Indium, Yttrium, lanthanides etc. The chelating ligand is preferably derived from ethylene diamine tetraacetic acid (EDTA), diethylene triamine pentaacetic acid (DTPA), cyclohexyl 1,2-diamine tetraaceticacid (CDTA), ethyleneglycol-O,O'-bis(2-aminoethyl)-N,N,N',N'-diacetic acid (HBED), triethylene tetraamine hexaacetic acid (TTHA), 1,4,7,10-tetraazacyclododecane-N,N',N"'-tetraacetic acid (DOTA), 1,4,7-triazacyclononane-N,N',N"-triacetic acid (NOTA), and 1,4,8,11-tetraazacyclotetradecane-N,N',N",N"'-tetraacetic acid (TETA), to either amine or thiol groups of the peptide compounds. The chelating ligands possess a suitable coupling group e.g. active esters, maleimides, thiocarbamates or α-halogenated acetamide moieties. For conjugating chelating ligands to amine groups e.g. ε-NH₂-groups of lysine residues previous reduction of the peptide compounds is not required. The radiolabeled peptides are suitable for radio-diagnostic and radio-therapeutic applications.

The resulting radiolabeled peptides show unexpected advantages in animal experiments. For example, excretion of a labeled peptide, e.g. ^{99m}Tc-labeled AP39 (Seq. Id. No. 11) in nude mice occurs to 70% or more, e.g. 80.63% within 24 hours via the kidneys, whereas for L19 (Seq. Id. No. 1) labeled with ¹²⁵I, excretion in nude mice occured only to 67.79% via the kidneys within 24 hours. The tumour to blood ratio of a labeled peptide, e.g. ^{99m}Tc-labeled AP39 is 5:1 or more, preferably 8:1 or more, e.g. about 10 : 1 after 5 hours, whereas for L19 labeled with ¹²⁵I, this ratio is only about 3 : 1. This is an unexpected behaviour also compared to other scFv antibodies labeled with ^{99m}Tc which often show less favourable biodistribution characteristics. For example, Verhaar et al., J. Nuc. Med., Vol. 37(5), pp. 868 - 872, 1996, report a ^{99m}Tc-labeled scFv antibody that shows a tumour to blood ratio of only 4 : 1 after 24 hours, and a kidney accumulation of 9% after 24 hours, which is very high compared to the values of the peptides described in the present invention, e.g. 1.3% for ^{99m}Tc-labeled AP39 (see example 13 below).

Further, the in vivo stability of the labeled peptides of the invention, e.g. ^{99m}Tc-labeled AP39 is much higher compared to the in vivo stability of L19 labeled with ¹²⁵I. The present inventors found that 2 hours after injection of a peptide, e.g. ^{99m}Tc-labeled AP39 only 10% or less, e.g. 3% of radioactivity within the serum was due to a metabolite, whereas 2 hours after injection of L19 labeled with ¹²⁵I, 49% of the radioactivity in the serum was due to metabolites, which may be free iodine. The improved *in vivo* stability of the peptides, e.g. ^{99m}Tc-labeled AP39 is also reflected by a prolonged preservation of its binding ability to the target ED-B. The present inventors found that 2 hours after injection of the peptide, e.g. ^{99m}Tc-labeled AP39, 50% or more, e.g. 74% of radioactivity within the serum was able to bind ED-B, whereas 2 hours after injection of I-125 labeled L19, only 27% of radioactivity within the serum could bind to ED-B. The compounds of this invention are also showing high tumour accumulation. Forexample, Tc-99m-AP39 and In-111-MX-DTPA-ε-HN(Lys)-AP39 displayed high tumour accumulation of 10.7 (Tc-99m ) or 12.9 (In-111) % injected dose per gram (ID/g) at 1 hour post injection (p.i.). Thus, tumor uptake is significantly higher compared to other known In-111 or Tc-99m labeled antibody fragments (e.g. Kobayashi et al., J. Nuc. Med., Vol. 41 (4), pp. 755 - 762, 2000; Verhaar et al., J. Nuc. Med., Vol. 37(5), pp. 868 - 872, 1996).

The compounds are suitable for diagnostic and therapeutic applications. They are preferably applied to the patient by parenteral administration, more preferably by intravenous injection. The human dose is preferably in the range of 0.1 to 1 mg per patient for radiodiagnostic applications, and 0.1 to 100 mg per patient for radiotherapeutic applications.

The methods for making and labeling the compounds of the present invention are more fully illustrated in the following examples. These examples are shown by way of illustration and not by way of limitation.

### Experimental

### Example 1: Production of L19 derivatives

A recombinant antibody (scFv L19, short name L19) against the extra domain B (ED-B) of a splice variant of fibronectin formed the starting material. scFv L19 had been isolated by means of phage display selection from a synthetic human antibody repertoire (Neri et al., 1997, Nature Biotechnol. 15: 1271; Pini et al., 1998, J. Biol. Chem. 273: 21769). This recombinant antibody fragment is in the form of a so-called single chain antibody fragment (scFv) and consists of a VH and VL region connected by a linker sequence (see Seq. Id. No. 1). This scFv L19 has exceptionally high affinity for ED B (K_{d}: 5.4 × 10⁻¹¹ M).

Various derivatives of L19 were produced by genetic manipulation (see Fig. 1). To modify L19, the scFv encoding DNA was amplified by PCR (polymerase chain reaction) using primers which coded for the additional sequences, and cloned into expression vectors.

**L19 derivatives:**

| | |
|---|---|
| L19: | without additional terminal modifications |
| L19 His: | C-terminal His₆ domain (His tag), for Ni chelate chromatography and for binding radioisotopes |
| AP38: | C-terminal GlyGlyGlyCys domain for binding (via Cys) substances which can be employed in therapy and diagnosis (e.g. radioisotopes) |
| AP39: | C-terminal GlyGlyGlyCysAla domain for binding (via Cys) substances which can be employed in therapy and diagnosis (e.g. radioisotopes) |
| L19-GlyCysGlyCys: | C-terminal GlyCysGlyCys domain for binding (via Cys) substances which can be employed in therapy and diagnosis (e.g. radioisotopes) |
| L19-GlyCysGlyCysAla: | C-terminal GlyCysGlyCysAla domain for binding (via Cys) substances which can be employed in therapy and diagnosis (e.g. radioisotopes) |

### Recombinant production of L19 derivatives

The L19 derivatives described were produced in prokaryotic and eukaryotic expression systems.

### a) L19 production in E. coli

The DNA sequences encoding various L19 derivatives (AP38, AP39, L19-GlyCysGlyCys, L19-GlyCysGlyCysAla, L19, L19His) were cloned into a prokaryotic expression vector (pDN5, Pini et al., 1997, J. Immunol. Methods 206: 171, Pini et al., 1998, J. Biol. Chem. 273: 21769; pET, Novagen) with IPTG-inducible promoter and ampicillin resistance marker. In order to make secretion of the recombinant protein into the periplasm possible, this vector was used to produce an expression cassette in which the N terminus of scFv is fused to a Pel B signal sequence. It was possible to establish stable producer strains by transforming E. coli (TG 1, BL21 DE3 and HB2151) with this expression vector, followed by ampicillin selection. To produce scFv, these strains were cultivated in the presence of 1 % glucose in the growth phase (37°C) in order to repress the promoter. Expression of scFv in the cultures was induced by adding IPTG and incubating at 30°C for up to 16 h. Soluble and antigen-binding scFv material could be isolated from the complete extract of the E. coli strains, from the periplasm fraction or, which proved to be particularly efficient in relation to purification and yield, from the culture supernatant. Production took place in shaken flasks and in fermenters with a culture volume of up to 10 litres.

### b) Production of L19 derivatives in Pichia pastoris

L19His, AP38, AP39, L19-GlyCysGlyCys and L19-GlyCysGlyCysAla-encoding DNA sequences were amplified by PCR and cloned into E. coli and into the expression vectors pPIC9K and pGAP (Invitrogen) for production in the yeast Pichia pastoris. For expression of heterologous genes, pPIC9K contains a methanol-inducible promoter (AOX1), and pGAP contains the constitutive promoter of the GAPDH enzyme. In addition, these vectors contain respectively a geneticin resistance gene and a zeocin resistance gene for selection/amplification of the foreign gene and a signal sequence (from yeast α factor) for expression and secretion of the recombinant product. The AP39 expression cassette used codes for a fusion protein (α factor signal + L19 derivatives) which contains for signal sequence elimination only a Kex2 cleavage site and not the other cleavage sites of natural α factor processing. Stable transfected PP clones were established by electroporation of the linearized vectors into Pichia pastoris strains (e.g. pPIC9K-AP39 into strain GS115, pGAP-AP39 into strain X33) and subsequent geneticin or zeocin selection. It was possible to use these clones to produce the said L19 derivatives as soluble secretory protein. The clones were cultivated at 30°C in BMGY medium or basal mineral medium. With clones based on pPIC, methanol was added for promoter induction during the expression phase. The recombinant product had a correctly processed terminus and high antigen-binding activity. The yields which could be achieved (unpurified, bioactive product/litre of culture supernatant) were, depending on the culturing conditions and process control: e.g. pPIC9K-AP39/GS115 (shaken flask 5 mg/l, fermenter 10-15 mg/l); pGAP-AP39/X33 (shaken flask 30-40 mg/l, fermenter 100-250 mg/l).

The L19 derivatives were purified from the Pichia pastoris or E. coli culture supernatant by use of affinity chromatography (rProtein A, Streamline Pharmacia or ED B antigen column) with subsequent size exclusion chromatography. The purified AP39 fraction, which was employed for further processing, had a homodimer structure (with subunits covalently linked for the most part) and high antigen-binding activity.

### Example 2a

### Synthesis of reduced AP38 [Reduced L19-(Gly)₃-Cyx-OH]

To a solution of 240 µg (4.29 nmol) S-S-dimeric AP38 in 156 µl PBS (phosphate buffered saline)/10% glycerine were added 50 µl TCEP-solution (14.34 mg TCEP x HCl/5 ml aqueous Na₂HPO₄, 0.1 M, pH = 7.4). The reaction mixture was gently shaken for 1 h at room temperature. SH-monomeric AP38 was purified by gel-chromatography using a NAP-5 column (Amersham, Eluent: PBS). SDS-PAGE analysis of the isolated product proofed the quantitative transformation of S-S-dimeric AP38 to SH-monomeric AP38.
Yield: 79.4 µg/220 µl PBS (33.1 %).

### Example 2b

### Synthesis of Tc-99m-AP38 [Tc-99m-L19-(Gly)₃-Cys-OH]

2.37 mg disodium-L-tartrate were placed in a vial followed by addition of 79.4 µg reduced AP38 in 220 µl PBS and the solution was diluted with 100 µl aqueous Na₂HPO₄-buffer (1 M, pH = 10.5). 50 µl Tc-99m generator eluate (24 h) and 10 µl SnCl₂-solution (5 mg SnCl₂/1 ml 0.1 M HCl) were added. The reaction mixture was shaken for 0.5 h at 37°C. Tc-99m-labeled AP38 was purified by gel-chromatography using a NAP-5 column (Amersham, Eluent: PBS).

| | |
|---|---|
| Radiochemical yield: | 39.7%. |
| Radiochemical purity: | 92.5% (SDS-PAGE). |
| Specific activity: | 17.7 MBq/nmol. |
| Immunoreactivity: | 88.7% |

### Example 3a

### Synthesis of reduced AP39 [Reduced L19-(Gly)₃-Cys-Ala-OH]

To a solution of 240 µg (4.29 nmol) S-S-dimeric AP39 in 135 µl PBS/10% glycerine were added 50 µl TCEP-solution (14.34 mg TCEP x HCl/5 ml aqueous Na₂HPO₄, 0.1 M, pH = 7.4). The reaction mixture was gently shaken for 1 h at room temperature. SH-monomeric AP39 was purified by gel-chromatography using a NAP-5 column (Amersham, Eluent: PBS). SDS-PAGE analysis of the isolated product proofed the quantitative transformation of S-S-dimeric AP39 to SH-monomeric AP39.
Yield: 135.9 µg/180 µl PBS (56.2%).

### Example 3b

### Synthesis of Tc-99m-AP39 [Tc-99m-L19-(Gly)₃-Cyc-Ala-OH]

4.2 mg disodium-L-tartrate were placed in a vial followed by addition of 135.9 µg reduced AP39 in 180 µl PBS and the solution was diluted with 100 µl aqueous Na₂HPO₄-buffer (1 M, pH = 10.5). 100 µl Tc-99m generator eluate (24 h) and 10 µl SnCl₂-solution (5 mg SnCl₂/1 ml 0.1 M HCl) were added. The reaction mixture was shaken for 0.5 h at 37°C. Tc-99m-labeled AP39 was purified by gel-chromatography using a NAP-5 column (Amersham, Eluent: PBS).

| | |
|---|---|
| Radiochemical yield: | 50.1%. |
| Radiochemical purity: | 91.5% (SDS-PAGE). |
| Specific activity: | 21.4 MBq/nmol. |
| Immunoreactivity: | 96.4% |

### Example 4

### Synthesis of Re-188-AP38 [Re-188-L19-(Gly)₃-Cys-OH]

2.37 mg disodium-L-tartrate were placed in a vial followed by addition of 112 µg reduced AP38 in 310 µl PBS and the solution was diluted with 100 µl aqueous Na₂HPO₄-buffer (1 M, pH = 10.5). 100 µl Re-188 generator eluate and 50 µl SnCl₂-solution (5 mg SnCl₂/1 ml 0.1 M HCl) were added. The reaction mixture was shaken for 1.5 h at 37°C. Re-188-labeled AP38 was purified by gel-chromatography using a NAP-5 column (Amersham, Eluent: PBS).

| | |
|---|---|
| Radiochemical yield: | 28.3%. |
| Radiochemical purity: | 91.1 % (SDS-PAGE). |
| Specific activity: | 15.3 MBq/nmol. |
| Immunoreactivity: | 89.9% |

### Example 5

### Synthesis of Re-188-AP39 [Re-188-L19-(Gly)₃-Cys-Ala-OH]

2.37 mg disodium-L-tartrate were placed in a vial followed by addition of 112 µg reduced AP39 in 303 µl PBS and the solution was diluted with 100 µl aqueous Na₂HPO₄-bufifer (1 M, pH = 10.5). 100 µl Re-188 generator eluate and 50 µl SnCl₂-solution (5 mg SnCl₂/1 ml 0.1 M HCl) were added. The reaction mixture was shaken for 1.5 h at 37°C. Re-188-labeled AP39 was purified by gel-chromatography using a NAP-5 column (Amersham, Eluent: PBS).

| | |
|---|---|
| Radiochemical yield: | 33.5%. |
| Radiochemical purity: | 92.3% (SDS-PAGE). |
| Specific activity: | 18.5 MBq/nmol. |
| Immunoreactivity: | 92.5% |

### Example 6a

### Synthesis of reduced L19-Gly-Cys-Gly-Cys-OH

To a solution of 240 µg (4.29 nmol) S-S-dimeric L19-Gly-Cys-Gly-Cys-OH in 160 µl PBS/10% glycerine were added 75 µl TCEP-solution (14.34 mg TCEP x HCl/5 ml aqueous Na₂HPO₄, 0.1 M, pH = 7.4). The reaction mixture was gently shaken for 1 h at room temperature. SH-monomeric L19-Gly-Cys-Gly-Cys-OH was purified by gel-chromatography using a NAP-5 column (Amersham, Eluent: PBS). SDS-PAGE analysis of the isolated product proofed the quantitative transformation of S-S-dimeric L19-Gly-Cys-Gly-Cys-OH to SH-monomeric L19-Gly-Cys-Gly-Cys-OH.
Yield: 80.4 µg/210 µl PBS (33.5%).

### Example 6b

### Synthesis of Tc-99m-L19-Gly-Cys-Gly-Cys-OH

2.37 mg disodium-L-tartrate were placed in a vial followed by addition of 80.4 µg reduced L19-Gly-Cys-Gly-Cys-OH in 210 µl PBS and the solution was diluted with 100 µl aqueous Na₂HPO₄-buffer (1 M, pH = 10.5). 50 µl Tc-99m generator eluate (24 h) and 10 µl SnCl₂-solution (5 mg SnCl₂/1 ml 0.1 M HCl) were added. The reaction mixture was shaked for 0.5 h at 37°C. Tc-99m-labeled L19-Gly-Cys-Gly-Cys-OH was purified by gel-chromatography using a NAP-5 column (Amersham, Eluent: PBS).

| | |
|---|---|
| Radiochemical yield: | 37.7%. |
| Radiochemical purity: | 91.5% (SDS-PAGE). |
| Specific activity: | 19.7 MBq/nmol. |
| Immunoreactivity: | 89.7% |

### Example 7a

### Synthesis of reduced L19-Gly-Cys-Gly-Cys-Ala-OH

To a solution of 240 µg (4.29 nmol) S-S-dimeric L19-Gly-Cys-Gly-Cys-Ala-OH in 155 µl PBS/10% glycerine were added 75 µl TCEP-solution (14.34 mg TCEP x HCl/5 ml aqueous Na₂HPO₄, 0.1 M, pH = 7.4). The reaction mixture was gently shaked for 1 h at room temperature. SH-monomeric L19-Gly-Cys-Gly-Cys-Ala-OH was purified by gel-chromatography using a NAP-5 column (Amersham, Eluent: PBS). SDS-PAGE analysis of the isolated product proofed the quantitative transformation of S-S-dimeric L19-Gly-Cys-Gly-Cys-Ala-OH to SH-monomeric L19-Gly-Cys-Gly-Cys-Ala-OH.
Yield: 81.2 µg/215 µl PBS (33.8%).

### Example 7b

### Synthesis of Tc-99m-L19-Gly-Cys-Gly-Cys-Ala-OH

2.37 mg disodium-L-tartrate were placed in a vial followed by addition of 81.2 µg reduced L19-Gly-Cys-Gly-Cys-Ala-OH in 215 µl PBS and the solution was diluted with 100 µl aqueous Na₂HPO₄-buffer (1 M, pH = 10.5). 50 µl Tc-99m generator eluate (24 h) and 10 µl SnCl₂-solution (5 mg SnCl₂/1 ml 0.1 M HCl) were added. The reaction mixture was shaked for 0.5 h at 37°C. Tc-99m-labeled L19-Gly-Cys-Gly-Cys-Ala-OH was purified by gel-chromatography using a NAP-5 column (Amersham, Eluent: PBS).

| | |
|---|---|
| Radiochemical yield: | 35.6%. |
| Radiochemical purity: | 93.5% (SDS-PAGE). |
| Specific activity: | 19.1 MBq/nmol. |
| Immunoreactivity: | 88.7% |

### Example 8a

### Synthesis of reduced AP39 for specific conjugation of EDTA, CDTA, TETA, DTPA, TTHA, HBED, DOTA, NOTA, and DO3A type chelators to the Cysteine-SH group

50µl TCEP-solution (14.34mg TCEPxHCl/5ml aqueous Na₂HPO₄, 0.1M, pH = 7.4) were added to a solution of 400µg (7.1 nmol) AP39 in 450µl PBS. The reaction mixture was gently shaken for 1 h at 37°C. Reduced AP39 was purified by gel-chromatography using a NAP-5 column (Amersham, Eluent: sodium acetate buffer, 0.1M, pH 5.0). SDS-PAGE analysis of the isolated product proofed the complete transformation of AP39 into reduced AP39.
Yield: 140µg/200µl (35%).

### Example 8b

### Synthesis of MX-DTPA-Maleimide (1,4,7-triaza-2-(N-maleimido ethylene p-amino)benzyl-1,7-bis(carboxymethyl)-4-carboxymethyl 6-methyl heptane)

512 mg (1 mmol) of {[3-(4-Amino-phenyl)-2-(bis-carboxymethyl-amino)-propyl]-[2-(bis-carboxymethyl-amino)-propyl]-amino}-acetic acid (Macrocyclics Inc. Dallas, TX, U.S.A.) and 707 mg (7 mmol) triethylamine were dissolved in 3 ml dry DMF. 400 mg (1,5 mmol) of 3-(2,5-Dioxo-2,5-dihydro-pyrrol-1-yl)-propionic acid 2,5-dioxo-pyrrolidin-1-yl ester (Aldrich) in 1 ml dry DMF were added dropwisely. The solution was stirred for 5 h at 50°C. 30 ml of diethylether were added slowly. The reaktion mixture was stirred for further 30 min. The principate was collected by filtering. The crude product was purified by RP-HPLC (acetonitrile- : water- : trifluoracetic acid / 3 : 96,9 : 0,1 → 99,9 : 0 : 0,1). Yield: 61 % (405 mg, 0,61 mmol). MS-ESI: 664 = M⁺ +1.

### Example 8c

### Synthesis of In-111-MX-DTPA-Maleimide-S(Cys)-AP39-R

### (R = reduced)

140 µg (5 nmol) AP39-R in 200 µl of sodium acetate buffer (0.1M, pH 5) were reacted with 50µl of dissolved 1,4,7-triaza-2-(N-maleimido ethylene p-amino)benzyl-1,7-bis(carboxymethyl)-4-carboxymethyl 6-methylheptane (0,25mg DTPA-Maleimide in 500µl sodium acetate buffer 0.1 M pH 5) for 3 h at 37°C. The reaction mixture was dialyzed 2 x 1 h with 200ml of sodium acetate buffer (0.1M, pH 6) employing a Slide-A-Lyzer 10,000 MWCO (Pierce Inc., Rockford, IL, U.S.A.).

80 µl [In-111]InCl₃ solution (HCl, 1 N, 40 MBq, Amersham Inc.) were added and the reaction mixture was heated at 37 °C for 30 min. In-111 labeled DTPA-Maleimide-S(Cys)-AP39-R was purified by gel-chromatography using a NAP-5 column (Amersham, Eluent: PBS).

| | |
|---|---|
| Radiochemical yield: | 54 %. |
| Radiochemical purity: | 94 % (SDS-PAGE). |
| Specific activity: | 6.2 MBq/nmol. |
| Immunoreactivity: | 86 % |

### Example 9

### Synthesis of In-111-MX-DTPA-∈-HN(Lys)-AP39

200 µg (3.6 nmol) non-reduced AP39 in 111 µl PBS were diluted with 300 µl of sodium borate buffer (0.1 M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 µl of 1,4,7-triaza-2-(p-isothiocyanato)benzyl-1,7-bis(carboxymethyl)-4-carboxymethyl-6-methyl heptane (MX-DTPA) solution (0.33 mg MX-DTPA dissolved in 500 µl of sodium borate buffer, 0.1 M, pH 8.5) were added and the reaction mixture was heated for 3 h at 37°C. The reaction mixture was dialyzed 2 x 1 h and 1 x 17 h (over night) with 200 ml of sodium acetate buffer (0.1 M, pH 6.0) each, employing the Slide-A-Lyzer 10,000 MWCO (Pierce Inc., Rockford, IL, U.S.A.).

80 µl [In-111] InCl₃ solution (HCl, 1 N, 40 MBq, Amersham Inc.) were added and the reaction mixture was heated at 37°C for 30 min. In-111 labeled MX-DTPA-∈-HN(Lys)-AP39 was purified by gel-chromatography using a NAP-5 column (Amersham, Eluent: PBS).

| | |
|---|---|
| Radiochemical yield: | 70 %. |
| Radiochemical purity: | 85 % (SDS-PAGE). |
| Specific activity: | 7.6 MBq/nmol. |
| Immunoreactivity: | 74 % |

### Example 10

### Synthesis of In-111 -DOTA-C-Benzyl-p-NCS -∈-HN(Lys)-AP39

200 µg (3.6 nmol) non-reduced AP39 in 114 µl PBS were diluted with 300 µl of sodium borate buffer (0.1 M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 µl of 1,4,7,10-tetraaza-2-(p-isothiocyanato)benzyl cyclododecane-1.,4,7,10-tetraacetic acid (benzyl-p-SCN-DOTA, Macrocyclics Inc., Dallas TX, U.S.A.) solution (1.5 mg benzyl-p-SCN-DOTA dissolved in 5 ml of sodium borate buffer, 0.1M, pH 8.5) were added to the solution and the reaction mixture was heated for 3 h at 37 °C. The reaction mixture was dialyzed 2 x 1 h and 1 x 17 h (over night) with 200 ml of sodium acetate buffer (0.1M, pH 6.0) each, employing the Slide-A-Lyzer 10,000 MWCO (Pierce Inc., Rockford, IL, U.S.A.).

80 µl [In-111]InCl₃ solution (HCl, 1 N, 40 MBq, Amersham Inc.) were added and the reaction mixture was heated at 37 °C for 30 min. In-111 labeled DOTA-C-Benzyl-p-NCS-∈-HN(Lys)-AP39 was purified by gel-chromatography using a NAP-5 column (Amersham, Eluent: PBS).

| | |
|---|---|
| Radiochemical yield: | 74 %. |
| Radiochemical purity: | 94 % (SDS-PAGE). |
| Specific activity: | 12.3 MBq/nmol. |
| Immunoreactivity: | 73 % |

### Example 11

### Synthesis of Y-88-MX-DTPA-∈-HN(Lys)-AP39

200 µg (3.6 nmol) non-reduced AP39 in 115 µl PBS were diluted with 300 µl of sodium borate buffer (0.1 M, pH 8.5) and dialyzed 2 x 1 h with 200ml of sodium borate buffer (0.1 M, pH 8.5) employing a Slide-A-Lyzer 10,000 MWCO (Pierce Inc., Rockford, IL, U.S.A.). 50 µl of MX-DTPA solution (0.33 mg MX-DTPA dissolved in 500 µl of sodium borate buffer, 0.1 M, pH 8.5) were added and the reaction mixture was heated for 3 h at 37°C. The reaction mixture was dialyzed 2 x 1 h and 1 x 17 h (over night) with 200 ml of sodium acetate buffer (0.1 M, pH 6.0) each, employing the Slide-A-Lyzer 10,000 MWCO (Pierce Inc., Rockford, IL, U.S.A.).

100 µl [Y-88]YCl₃ solution (HCl, 1 N, 75 MBq, Oak Ridge National Lab.) were added and the reaction mixture was heated at 37°C for 30 min. Y-88 labeled MX-DTPA-∈-HN(Lys)-AP39 was purified by gel-chromatography using a NAP-5 column (Amersham, Eluent: PBS).

| | |
|---|---|
| Radiochemical yield: | 65 %. |
| Radiochemical purity: | 93 % (SDS-PAGE). |
| Specific activity: | 10.2 MBq/nmol. |
| Immunoreactivity: | 72 % |

### Example 12

### Synthesis of Lu-177 -DOTA-C-Benzyl-p-NCS-ε-HN(Lys)-AP3

200 µg (3.6 nmol) non-reduced AP39 in 110 µl PBS were diluted with 300 µl of sodium borate buffer (0.1M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 µl of benzyl-p-SCN-DOTA solution (1.5 mg dissolved in 5 ml of sodium borate buffer, 0.1M, pH 8.5) were added and the reaction mixture was heated for 3 h at 37°C. The reaction mixture was dialyzed 2 x 1 h and 1 x 17 h (over night) with 200 ml of sodium acetate buffer (0.1 M, pH 6.0) each, employing the Slide-A-Lyzer 10,000 MWCO (Pierce Inc., Rockford, IL, U.S.A.).

200 µl [Lu-1771]LuCl3 solution (HCl, 1 N, 80 MBq, NRH-Petten, Netherlands) were added and the reaction mixture was heated at 37°C for 30 min. Lu-177 labeled DOTA-C-Benzyl-p-NCS-∈-HN(Lys)-AP39 was purified by gel-chromatography using a NAP-5 column (Amersham, Eluent: PBS).

| | |
|---|---|
| Radiochemical yield: | 74 %. |
| Radiochemical purity: | 95 % (SDS-PAGE). |
| Specific activity: | 19 MBq/nmol. |
| Immunoreactivity: | 71 % |

### Example 13

### Organ distribution and excretion of Tc-99m-AP39, expressed in Pichia pastoris, after a single i.v. injection into tumour-bearing nude mice

The substance of the invention is injected intravenously in a dose of about 74 kBq into F9 (teratocarcinoma)-bearing animals (bodyweight about 25 g). The radioactivity concentration in various organs, and the radioactivity in the excreta are measured using a *Y* counter at various times after administration of the substance. In addition, the tumour to blood ratio is found at various times on the basis of the concentration of the substance of the invention in tumour and blood.

The biodistribution of Tc-99m-AP39 in F9 (teratocarcinoma)-bearing nude mice (mean ± SD, n =3) is shown in Table 2:

**Table 2**

| | % of dose / g of tissue | % of dose / g of tissue | % of dose / g of tissue |
|---|---|---|---|
| | 1 h p.i. | 5 h p.i. | 24 h p.i. |
| Spleen | 1.97 ± 0.018 | 0.53 ±0.07 | 0.31 ± 0.08 |
| Liver | 1.91 ± 0.046 | 0.77 ±0.07 | 0.26 ±0.01 |
| Kidney | 19.21 ±0.70 | 4.35 ±0.082 | 1.32 ±0.10 |
| Lung | 3.43 ± 1.01 | 1.41 ±0.032 | 0.96 ±0.23 |
| Stomach without contents | 1.55 ±0.043 | 1.35 ±0.22 | 0.48 ±0.10 |
| Intestine with contents | 1.42 ±0.10 | 1.26 ±0.34 | 0.29 ±0.05 |
| Tumour | 10.72 ±3.21 | 5.13 ±1.45 | 3.48 ±1.28 |
| Blood | 3.03 ±0.32 | 0.57 ±0.1 | 0.11 ±0.01 |

The excretion of Tc-99m-AP39 in F9 (teratocarcinoma)-bearing nude mice (mean ± SD, n = 3) is shown in Table 3:

**Table 3**

| | % of dose |
|---|---|
| | 24 h p.i. |
| Urine | 80.63 ±3.33 |
| Faeces | 3.94 ±0.17 |

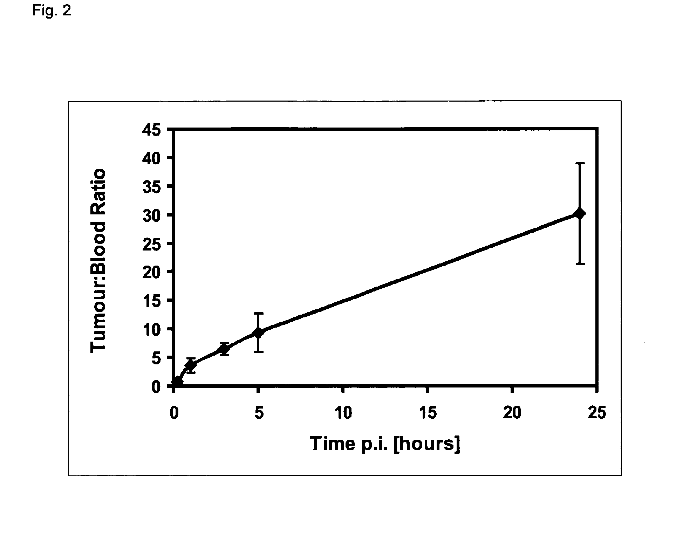

The tumour to blood ratio of Tc-99m-AP39 in F9 (teratocarcinoma)-bearing nude mice (mean ± SD, n = 3) is shown in Fig. 2.

The results of this investigation show the excellent potential of the substance of the invention for accumulation in solid tumours with, at the same time, excellent excretion.

### Example 14

### Organ distribution of In-111-MX-DTPA-∈-HN(Lys)-AP39 after a single i.v. injection into tumour-bearing nude mice

The substance of the invention is injected intravenously in a dose of about 48 kBq into F9 (teratocarcinoma)-bearing animals (body weight about 25 g). The radioactivity concentration in various organs, and the radioactivity in the excreta are measured using a *Y* counter at various times after administration of the substance.

The biodistribution of In-111-MX-DTPA-∈-HN(Lys)-AP39 in F9 (teratocarcinoma)-bearing nude mice (mean ± SD, n=3) is shown in Table 4:

**Table 4**

| | % dose / g of tissue | | |
|---|---|---|---|
| | 1h p.i. | 3h p.i. | 24h p.i. |
| Spleen | 1.94 ± 0.49 | 1.28 ± 0.13 | 1.18 ± 0.24 |
| Liver | 2.61 ± 1.32 | 2.59 ± 0.36 | 2.26 ± 0.75 |
| Lung | 1.52 ± 1.57 | 2.36 ± 0.30 | 0.76 ± 0.21 |
| Stomach without contents | 1.44 ± 0.81 | 1.40 ± 0.31 | 0.65 ± 0.28 |
| Intestine with contents | 5.05 ± 5.26 | 1.07 ± 0.34 | 0.67 ± 0.11 |
| Tumour | 12.90 ± 4.81 | 7.44 ± 1.34 | 4.33 ± 0.84 |
| Blood | 5.55 ± 1.89 | 1.80 ± 0.20 | 0.11 ± 0.02 |

The tumour to blood ratio of In-111-MX-DTPA-∈-HN(Lys)-AP39 in F9 (teratocarcinoma)-bearing nude mice (mean ± SD, n=3) is shown in Table 5.

**Table 5**

| | 1h p.i. | 3h p.i. | 24h p.i. |
|---|---|---|---|
| Tumour to blood ratio | 2.76 ± 2.00 | 4.16 ± 0.75 | 36.36 ± 3.78 |

The results of this investigation show the excellent potential of the substance of the invention for accumulation in solid tumours paired with excellent biodistribution and tumor to blood ratio.

### Example 15 Imaging of Tc-99m-AP39, expressed in Pichia pastoris, after a single i.v. injection into tumour-bearing nude mice

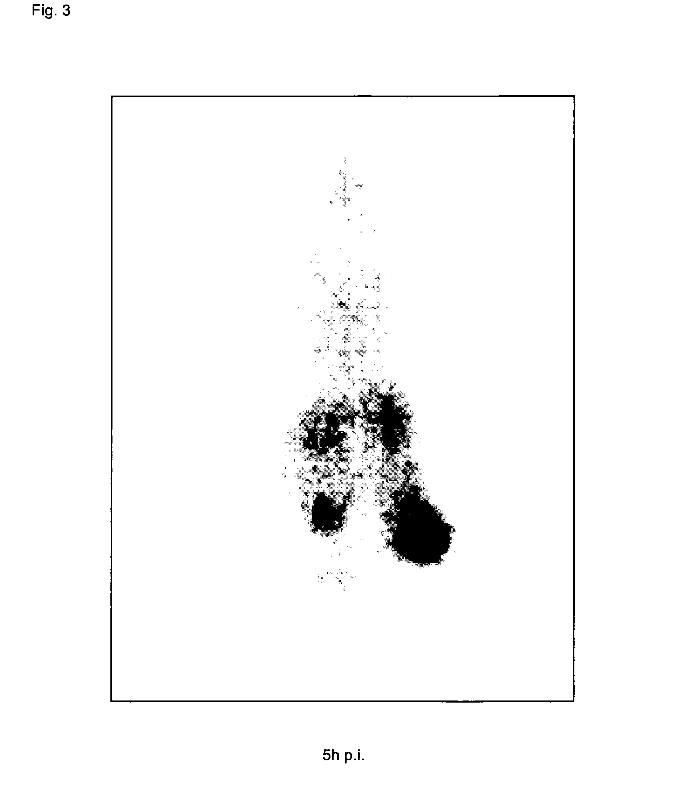

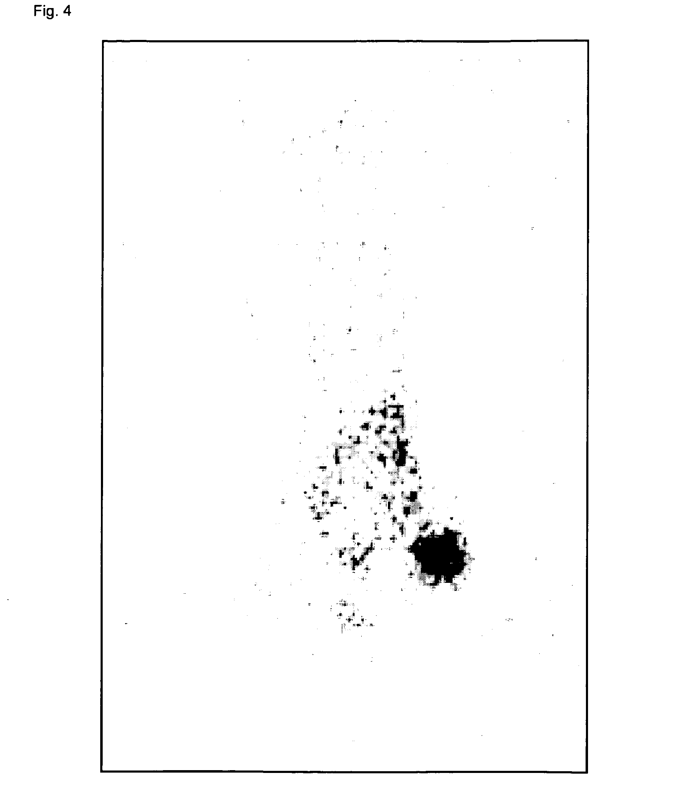

The substance of the invention is injected intravenously in a dose of about 9.25 MBq into F9 (teratocarcinoma)-bearing animals (bodyweight about 25 g). Gamma-camera imaging is carried out at various times after administration of the substance. Planar scintigraphy of Tc-99m-AP39 in F9 (teratocarcinoma)-bearing nude mice is shown in Figures 3 and 4. Fig. 3 shows the scintigram 5 hours after injection of the substance, and Fig. 4 shows the scintigram 24 hours after injection of the substance.

The result of this investigation shows the excellent potential of the substance of the invention for imaging solid tumours.

Further, the invention is described by the following items:
1. A compound comprising
   a peptide comprising
   - aa): an antigen-binding site for the extra domain B (ED-B) of fibronectin comprising complementarity-determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to Seq. Id. No. 1,
   - ab): an antigen-binding site for the extra domain B (ED-B) of fibronectin comprising complementarity-determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to Seq. Id. No. 1, or
   - ac): a sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
   and
   - ba): an amino acid sequence Xaa₁-Xaa₂₋Xaa₃-Cys (Seq. Id. No. 2), wherein Xaa₁, Xaa₂ and Xaa₃ each independently represent any naturally occuring amino acid, or
   - bb): an amino acid sequence Xaa₁-Xaa₂-Xaa₃-Cys-Xaa₄ (Seq. Id. No. 3), wherein Xaa₁, Xaa₂, Xaa₃, and Xaa₄ each independently represent any naturally occuring amino acid,
   - bc): or an amino acid sequence (His)ₙ (Seq. Id. No. 4), wherein n stands for an integer from 4 to 6,
   wherein the C-terminus of aa), ab) or ac) is bound to the N-terminus of one of the sequences Seq. Id. No. 2, Seq. Id. No. 3 or Seq. Id. No. 4 via a peptide bond.
2. The compound according to item 1, wherein the amino acid sequence Xaa₁-Xaa₂-Xaa₃-Cys (Seq. Id. No. 2) is the sequence Gly-Gly-Gly-Cys (Seq. Id. No. 5) or Gly-Cys-Gly-Cys (Seq. Id. No. 6).
3. The compound according to item 1, wherein the amino acid sequence Xaa₁-Xaa₂-Xaa₃-Cys-Xaa₄ (Seq. Id. No. 3) is the sequence Gly-Gly-Gly-Cys-Ala (Seq. Id. No. 7) or Gly-Cys-Gly-Cys-Ala (Seq. Id. No. 8).
4. The compound according to item 1, wherein n in the amino acid sequence (His)ₙ (Seq. Id. No. 4) is 6.
5. The compound according to any one of items 1-4, which is conjugated to a radioisotope.
6. The compound according to item 5, which is conjugated to a radioisotope selected from a radioisotope of Technetium, such as ^{94m}Tc, ^{99m}Tc, Rhenium, such as ¹⁸⁶Re, ¹⁸⁸Re, or other isotopes, such as ²⁰³Pb, ⁶⁷Ga, ⁶⁸Ga, ⁴³Sc, ⁴⁴Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ⁷²As and ¹⁸F.
7. A compound according to item 6, wherein the radioisotope is ^{99m}Tc or ¹⁸⁸Re.
8. The compound according to any one of items 1-7, wherein the peptide is in reduced form.
9. A pharmaceutical composition comprising as an active agent a compound according to any one of items 1-8 together with physiologically acceptable adjuvants, carriers and/or diluents.
10. The composition of item 9, which is excreted to 70 % or more via the kidneys within 24 hours in mice.
11. The composition of item 9 or 10 having a tumour to blood ratio of 5:1 or more 5 h after administration in mice.
12. The composition of any one of item 9-11 for diagnostic applications.
13. The composition of any one of items 9-11 for therapeutic applications.
14. Use of a peptide comprising
   - aa): an antigen-binding site for the extra domain B (ED-B) of fibronectin comprising complementarity-determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to Seq. Id. No. 1,
   - ab): an antigen-binding site for the extra domain B (ED-B) of fibronectin comprising complementarity-determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to Seq. Id. No. 1, or
   - ac): a sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
   and
   - ba): an amino acid sequence Xaa₁-Xaa₂-Xaa₃-Cys (Seq. Id. No. 2), wherein Xaa₁, Xaa₂ and Xaa₃ each independently represent any naturally occuring amino acid or
   - bb): an amino acid sequence Xaa₁-Xaa₂-Xaa₃-Cys-Xaa₄ (Seq. Id. No. 3), wherein Xaa₁, Xaa₂, Xaa₃, and Xaa₄ each independently represent any naturally occuring amino acid or
   - bc): an amino acid sequence (His)ₙ (Seq. Id. No. 4), wherein n stands for an integer from 4 to 6, wherein the C-terminus of aa), ab) or ac) is bound to the N- terminus of one of the sequences Seq. Id. No. 2, Seq. Id. No. 3 or Seq. Id. No. 4 via a peptide bond, for binding a radioisotope.
15. The use according to item 14, wherein the radioisotope is selected from a radioisotope of Technetium, such as ^{94m}Tc, ^{99m}Tc, Rhenium, such as ¹⁸⁶Re, ¹⁸⁸Re, or other isotopes, such as ²⁰³Pb, ⁶⁷Ga, ⁶⁸Ga, ⁴³Sc, ⁴⁴Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ⁷²As and ¹⁸F.
16. The use according to item 15, wherein the radioisotope is ^{99m}Tc or ¹⁸⁸Re.
17. A process for the production of a peptide as defined in any one of items 1-4, **characterized in that** the peptide is expressed in eukaryotic cells, particularly in yeast cells.
18. The process according to item 17, wherein the eukaryotic cells are *Pichia pastoris* cells.
19. The process according to item 17 or 18, wherein the peptide is expressed constitutively.
20. The process according to any one of items 17-19, wherein the N-terminus of the peptide is directly fused to the Kex2-cleavage site from the α-signal sequence.
21. A kit for the production of radiopharmaceuticals comprising a peptide as defined in any one of items 1-8, optionally together with physiologically acceptable adjuvants.

## Claims

1. A compound comprising
a peptide comprising
aa) an antigen-binding site for the extra domain B (ED-B) of fibronectin comprising complementarity-determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to Seq. Id. No. 1,
ab) an antigen-binding site for the extra domain B (ED-B) of fibronectin comprising complementarity-determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to Seq. Id. No. 1, or
ac) a sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
and
ba) an amino acid sequence Xaa₁-Xaa₂-Xaa₃-Cys (Seq. Id. No. 2), wherein Xaa₁, Xaa₂ and Xaa₃ each independently represent any naturally occuring amino acid, or
bb) an amino acid sequence Xaa₁-Xaa₂-Xaa₃-Cys-Xaa₄(Seq. Id. No. 3), wherein Xaa₁, Xaa₂, Xaa₃, and Xaa₄ each independently represent any naturally occuring amino acid, or
bc) an amino acid sequence (His)ₙ (Seq. Id. No. 4), wherein n stands for an integer from 4 to 6, in particular 6, wherein the C-terminus of aa), ab) or ac) is bound to the N-terminus of one of the sequences Seq. Id. No. 2, Seq. Id. No. 3 or Seq. Id. No. 4 via a peptide bond.

2. The compound according to claim 1, wherein the amino acid sequence Xaa₁-Xaa₂-Xaa₃-Cys (Seq. Id. No. 2) is the sequence Gly-Gly-Gly-Cys (Seq. Id. No. 5) or Gly-Cys-Gly-Cys (Seq. Id. No. 6).

3. The compound according to claim 1, wherein the amino acid sequence Xaa₁-Xaa₂-Xaa₃-Cys-Xaa₄ (Seq. Id. No. 3) is the sequence Gly-Gly-Gly-Cys-Ala (Seq. Id. No. 7) or Gly-Cys-Gly-Cys-Ala (Seq. Id. No. 8).

4. The compound according to any one of claims 1-3, which is conjugated to a radioisotope.

5. The compound according to claim 4, which is conjugated to a radioisotope selected from a radioisotope of Technetium, such as ^{94m}Tc, ^{99m}Tc, Rhenium, such as ¹¹⁶Re, ¹⁸⁸Re, or other isotopes, such as ²⁰³Pb, ⁶⁷Ga, ⁶⁸Ga, ⁴³Sc, ⁴⁴Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ⁷²As and ¹⁸F, in particular preferably ^{99m}Tc or ¹⁸⁸Re.

6. The compound according to any one of claims 1-5, wherein the peptide is in reduced form.

7. A pharmaceutical composition comprising as an active agent a compound according to any one of claims 1-6 together with physiologically acceptable adjuvants, carriers and/or diluents.

8. The composition of claim 7, which is excreted to 70 % or more via the kidneys within 24 hours in mice.

9. The composition of claim 7 or 8 having a tumour to blood ratio of 5:1 or more 5 h after administration in mice.

10. The composition of any one of claims 7-9 for diagnostic applications or therapeutic applications.

11. Use of a peptide comprising
aa) an antigen-binding site for the extra domain B (ED-B) of fibronectin comprising complementarity-determining regions HCDR3 and/or LCDR3 as shown in Table 1 or a variation thereof that is a deletion, insertion and/or substitution of up to 5 amino acids for the HCDR3 region and up to 6 amino acids for the LCDR3 region, which has the same function as a peptide according to Seq. Id. No. 1,
ab) an antigen-binding site for the extra domain B (ED-B) of fibronectin comprising complementarity-determining regions HCDR1, HCDR2, HCDR3, LCDR1, LCDR2 and LCDR3 as shown in Table 1 or a variation thereof that is a deletion, insertion and/or substitution of up to 3 amino acids for the HCDR1 region, up to 8 amino acids for the HCDR2 region, up to 5 amino acids for the HCDR3 region, up to 6 amino acids for the LCDR1 region, up to 4 amino acids for the LCDR2 region and up to 6 amino acids for the LCDR3 region, which has the same function as a peptide according to Seq. Id. No. 1, or
ac) a sequence according to Seq. Id. No. 1 (L19) or a variation of Seq. Id. No. 1 that is a deletion, insertion and/or substitution of up to 30 amino acids, and which has the same function as a peptide according to Seq. Id. No. 1,
and
ba) an amino acid sequence Xaa₁-Xaa₂-Xaa₃-Cys (Seq. Id. No. 2), wherein Xaa₁, Xaa₂ and Xaa₃ each independently represent any naturally occuring amino acid or
bb) an amino acid sequence Xaa₁-Xaa₂-Xaa₃-Cys-Xaa₄(Seq. Id. No. 3), wherein Xaa₁, Xaa₂, Xaa₃, and Xaa₄ each independently represent any naturally occuring amino acid or
bc) an amino acid sequence (His)ₙ (Seq. Id. No. 4), wherein n stands for an integer from 4 to 6, wherein the C-terminus of aa), ab) or ac) is bound to the N- terminus of one of the sequences Seq. Id. No. 2, Seq. Id. No. 3 or Seq. Id. No. 4 via a peptide bond, for binding a radioisotope.

12. The use according to claim 11, wherein the radioisotope is selected from a radioisotope of Technetium, such as ^{94m}Tc, ^{99m}Tc, Rhenium, such as ¹⁸⁶Re, ¹⁸⁸Re, or other isotopes, such as ²⁰³Pb, ⁶⁷Ga, ⁶⁸Ga, ⁴³Sc, ⁴⁴Sc, ⁴⁷Sc, ^{110m}In, ¹¹¹In, ⁹⁷Ru, ⁶²Cu, ⁶⁴Cu, ⁶⁷Cu, 68Cu, ⁸⁶Y, ⁸⁸Y, ⁹⁰Y, ¹²¹Sn, ¹⁶¹Tb, ¹⁵³Sm, ¹⁶⁶Ho, ¹⁰⁵Rh, ¹⁷⁷Lu, ⁷²As and ¹⁸F, in particular preferably ^{99m}Tc or ¹⁸⁸Re.

13. A process for the production of a peptide as defined in any one of claims 1-4, **characterized in that** the peptide is expressed, preferably constitutively, in eukaryotic cells, particularly in yeast cells, most preferably Pichia pastoris cells.

14. The process according to claim 13, wherein the N-terminus of the peptide is directly fused to the Kex2-cleavage site from the α-signal sequence.

15. A kit for the production of radiopharmaceuticals comprising a peptide as defined in any one of claims 1-6, optionally together with physiologically acceptable adjuvants.


SEQUENCE LISTING

<110> Bayer Schering Pharma Aktiengesellschaft

<120> New methods for diagnosis and treatment of tumours

<130> 27041P EP-WO-1

<140>
<141>

<150> EP02 000 315.8
<151> 2002-01-03

<150> US60/358702
<151> 2002-02-25

<160> 13

<170> PatentIn Ver. 2.1

<210> 1
<211> 238
<212> PRT
<213> Artificial Sequence

<220>
<221> SITE
<222> (1)..(116)
<223> VH

<220>
<221> SITE
<222> (117)..(130)
<223> Linker

<220>
<221> SITE
<222> (131)..(238)
<223> VL

<220>
<221> VARIANT
<222> (1)..(238)
<223> deletion, insertion and/or substitution of up to
      30 amino acids

<220>
<223> Description of Artificial Sequence: recombinant
      antibody fragment

<400> 1
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125


    130                 135                 140


145                 150                 155                 160


                165                 170                 175


            180                 185                 190


        195                 200                 205


    210                 215                 220


225                 230                 235


<210> 2
<211> 4
<212> PRT
<213> Artificial Sequence

<220>
<223> Description of Artificial Sequence: recombinant
      antibody fragment

<220>
<221> VARIANT
<222> (1)..(3)
<223> xaa each independently represent any naturally
      occuring amino acid

<400> 2
Xaa Xaa Xaa Cys
  1


<210> 3
<211> 5
<212> PRT
<213> Artificial Sequence

<220>
<223> Description of Artificial Sequence: recombinant
      antibody fragment

<220>
<221> VARIANT
<222> (1)..(3)

<223> Xaa each independently represent any naturally
      occuring amino acid

<220>
<221> VARIANT
<222> (5)
<223> Xaa represents any naturally occuring amino acid

<400> 3
Xaa Xaa Xaa Cys Xaa
  1               5



<210> 4
<211> 1
<212> PRT
<213> Artificial Sequence

<220>
<223> Description of Artificial Sequence: recombinant
      antibody fragment

<220>
<221> REPEAT
<222> (1)
<223> His=(His)n, wherein n stands for an integer from 4
      to 6

<400> 4
His
  1



<210> 5
<211> 4
<212> PRT
<213> Artificial Sequence

<220>
<223> Description of Artificial Sequence: recombinant
      antibody fragment

<400> 5
Gly Gly Gly Cys
  1



<210> 6
<211> 4
<212> PRT
<213> Artificial Sequence

<220>
<223> Description of Artificial Sequence: recombinant
      antibody fragment

<400> 6
Gly Cys Gly Cys
  1



<210> 7

<211> 5
<212> PRT
<213> Artificial Sequence

<220>
<223> Description of Artificial Sequence: recombinant
      antibody fragment

<400> 7
Gly Gly Gly Cys Ala
  1               5

<210> 8
<211> 5
<212> PRT
<213> Artificial Sequence

<220>
<223> Description of Artificial Sequence: recombinant
      antibody fragment

<400> 8
Gly Cys Gly Cys Ala
  1               5

<210> 9
<211> 247
<212> PRT
<213> Artificial Sequence

<220>
<223> Description of Artificial Sequence: recombinant
      antibody fragment

<400> 9
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Ala Ala Ala Leu
225                 230                 235                 240

Glu His His His His His His
                245

<210> 10
<211> 240
<212> PRT
<213> Artificial Sequence

<220>
<223> Description of Artificial Sequence: recombinant
      antibody fragment

<400> 10
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Gly Gly Gly Cys
225                 230                 235                 240

<210> 11
<211> 241
<212> PRT
<213> Artificial Sequence

<220>
<223> Description of Artificial Sequence: recombinant
      antibody fragment

<400> 11
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Gly Gly Gly Cys
225                 230                 235                 240

Ala

<210> 12
<211> 240
<212> PRT
<213> Artificial Sequence

<220>
<223> Description of Artificial Sequence: recombinant
      antibody fragment

<400> 12
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Gly Cys Gly Cys
225                 230                 235                 240

<210> 13
<211> 241
<212> PRT
<213> Artificial Sequence

<220>
<223> Description of Artificial Sequence: recombinant
      antibody fragment

<400> 13
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Ser Phe
             20                  25                  30

Ser Met Ser Trp Val Arg Gln Ala Pro Gly Lys Gly Leu Glu Trp Val
         35                  40                  45

Ser Ser Ile Ser Gly Ser Ser Gly Thr Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Lys Pro Phe Pro Tyr Phe Asp Tyr Trp Gly Gln Gly Thr Leu Val
            100                 105                 110

Thr Val Ser Ser Gly Asp Gly Ser Ser Gly Gly Ser Gly Gly Ala Ser
        115                 120                 125

Glu Ile Val Leu Thr Gln Ser Pro Gly Thr Leu Ser Leu Ser Pro Gly
    130                 135                 140

Glu Arg Ala Thr Leu Ser Cys Arg Ala Ser Gln Ser Val Ser Ser Ser
145                 150                 155                 160

Phe Leu Ala Trp Tyr Gln Gln Lys Pro Gly Gln Ala Pro Arg Leu Leu
                165                 170                 175

Ile Tyr Tyr Ala Ser Ser Arg Ala Thr Gly Ile Pro Asp Arg Phe Ser
            180                 185                 190

Gly Ser Gly Ser Gly Thr Asp Phe Thr Leu Thr Ile Ser Arg Leu Glu
        195                 200                 205

Pro Glu Asp Phe Ala Val Tyr Tyr Cys Gln Gln Thr Gly Arg Ile Pro
    210                 215                 220

Pro Thr Phe Gly Gln Gly Thr Lys Val Glu Ile Lys Gly Cys Gly Cys
225                 230                 235                 240

Ala